# EUROPEAN PATENT SPECIFICATION

(11) **EP 1 787 730 B1**
(45) Date of publication and mention of the grant of the patent: **11.05.2016**
(21) Application number: 06123870.5
(22) Date of filing: 10.11.2006
(51) Int. Cl.: B08B 3/04, F26B 15/14

(54) **Apparatus for moving planes for washing chambers**
Vorrichtung zur Bewegung von Platten in einem Waschraum
Appareil pour déplacer des plateaux dans une enceinte de lavage

(30) Priority: 16.11.2005 IT UD20050193
(43) Date of publication of application: 23.05.2007
(73) Proprietor: STEELCO SPA, 31039 Riese Pio X (IT)
(72) Inventor: Zardini, Fabio, 31033, Castelfranco Veneto (TV) (IT)
(74) Representative: Petraz, Gilberto Luigi

(56) References cited:
- DE-A1- 3 545 867
- US-A- 1 551 305
- US-A- 6 021 790

## Description

### FIELD OF THE INVENTION

The present invention concerns an apparatus to move planes for washing chambers such as for example those used in hospitals for washing trolley-mounted objects, trolleys, beds, bedside tables or other equipment to be washed and/or sterilized.

### BACKGROUND OF THE INVENTION

Washing chambers are known, used for washing re-usable equipment in contexts such as hospitals, or laboratories, for example trolleys, beds and suchlike. Such washing chambers, for example those of the hospital type, usually include a grid-type plane or surface so that the water can flow away, on which the objects to be subjected to the washing cycle and heat disinfection rest.

The washing and heat disinfection cycle is carried out inside the washing chamber, by means of jets of hot water and/or steam, with the aid of suitable detergents.

In particular, the washing water and/or steam is delivered preferentially in several successive washing steps, which always take place inside the washing chamber, for example at different temperatures, flow rate, pressure, geometry of delivery and similar parameters.

At the end of each washing step, the objects washed, which are of the most disparate shape, often have a large quantity of washing water, left over from the washing cycle just completed, on their horizontal surfaces.

The presence of the residual washing water is a disadvantage which requires manual intervention by several operators, also because of the considerable sizes of the objects washed, in order to discharge the water, usually at the end of every washing step or at the end of the whole washing cycle, with a consequent delay in completing the washing cycle and large involvement of workers.

Moreover, the water stagnating at the end of every washing step is often rinsing water, and therefore with a high concentration of residual detergent, or it is water that has impurities, dirt and various residues: therefore, on the surfaces where the residual water stagnates, the hygienic-sanitary conditions required by the places which use said washing chambers are not guaranteed.

Apparatuses are known for inclining the loading plane of a washing chamber, provided with an inclination device with multiple pistons, with a fluid-dynamic drive, for example oil-dynamic or pneumatic, and able to be activated individually in a selective and alternating manner, in order to incline the plane of the washing chamber in selected directions and thus allow both the complete discharge of the residual water, and also the facilitated discharge of the objects from the washing chamber.

Examples of known inclination apparatuses used to incline the loading plane of a washing chamber are described in US-A-1-551-305, which is considered as the closest prior art, or DE-A-3-545-867 or US-A-6-021-790.

However, these known apparatuses require a complex drive system, in which each piston is associated with its own motor, and in which moreover the movement of one piston must be synchronized with the other pistons, so as to achieve the desired inclination, by means of an elaborate and costly automatic control.

Purpose of the invention is to achieve an apparatus to move planes for washing chambers, for example used in the industrial and/or hospital field, which allows to effect the automatic discharge of the residual water from the objects washed, inside the washing chamber, and also facilitates the discharge of the objects from the washing chamber, in an effective and economical manner, without needing a costly and complex inclination and actuation system.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

According to the present invention, the movement apparatus is able to selectively influence the position of a loading plane of a washing chamber mainly, but not only, in order to allow the washing water to flow away from the horizontal surfaces of the objects to be washed, such as trolley-mounted objects like a trolley, a bed and other, disposed on said loading plane.

The loading plane is advantageously made of stainless steel or other stainless materials such as titanium, plastic and suchlike, and advantageously has a grid-like structure, to allow the washing water to flow away.

The loading plane is preferentially provided with two lanes, substantially parallel, on which an object to be washed is able to slide, for example provided with sliding means such as castor-type wheels and suchlike, such as a trolley, a bed, a bedside table. The loading plane is not solidly constrained to the floor of the washing chamber, so that it is freely inclinable by means of the movement apparatus according to the present invention.

Thanks to the loading plane with two lanes, having transverse sizes substantially analogous to the section of the sliding means of the object to be washed, allows the jets of water arriving from below to hit the maximum surface possible of the objects resting thereon.

Moreover, the structure with lanes is preferred because it allows a considerable reduction in the material used, with a simultaneous reduction in the weight and costs of the loading plane. The lanes, moreover, advantageously direct the object towards the exit, once washing is terminated.

In order to effect the wash, the object to be washed is put on the loading plane, inside the washing chamber, and subsequently the washing and heat disinfection cycle takes place.

To this purpose, the washing chamber is provided with nozzles suitable to emit hot water and/or steam, advantageously so as to hit the objects to be washed substantially from all directions and optionally in association with specific detergent and/or disinfecting products.

According to the present invention, the movement apparatus comprises an inclination device positioned between the floor of the washing chamber and the loading plane. The inclination device is provided with at least a first and a second rotation bar, distanced from each other over the length of the loading plane and advantageously parallel with each other and transverse to the direction of movement of the objects to be washed. The rotation bars are able to be rotated by means of a single drive system, or motor, which allows them to rotate around their own axis. The first and second rotation bars are provided with respective first and second cam elements, able to interfere with the loading plane when the rotation bars are rotated clock-wise or anti-clockwise, so as to raise and incline the loading plane and consequently vary the inclination of the objects resting thereon, in at least two transverse directions, that is, a first direction of inclination to discharge the water and a different second direction of inclination to discharge the object. According to a characteristic feature of the present invention, the first and second cam elements are disposed on the respective rotation bars, so that the rotation thereof in one direction determines the first inclination on one side of the loading plane in order to discharge the residual washing water from the surfaces of the objects to be washed; on the contrary, the rotation of at least one bar in the opposite direction to the previous one determines a second inclination forwards of the loading plane, that is, towards the exit, with respect to the direction in which the objects are inserted into the washing chamber, in order to facilitate the discharge of the objects from the washing chamber. In this way, with a single motor and hence a single automatism, the two rotation bars are made to rotate simultaneously and the selective and desired inclination of the loading plane is obtained according to the washing step in progress. Advantageously, when the loading plane is inclined in order to discharge the water, the object is kept in position by the interference of the lanes with the sliding means of the object itself, whereas, on the contrary, when the loading plane is inclined towards the exit, the lanes allow the object to slide freely outside the washing chamber.

### BRIEF DESCRIPTION OF THE DRAWINGS

These and other characteristics of the present invention will become apparent from the following description of a preferential form of embodiment, given as a non-restrictive example with reference to the attached drawings wherein:
- - fig. 1: is a front schematic view of a washing chamber provided with an apparatus to move planes for washing chambers according to the present invention shown with a continuous line in an inactive position and with a line of dashes in an inclined position;
- - fig. 2: is a lateral schematic view of the washing chamber in fig. 1, with the apparatus to move planes for washing chambers according to the present invention shown with a continuous line in an inactive position and with a line of dashes in another inclined position;
- - fig. 3: is a perspective view of an apparatus to move planes for washing chambers in an inactive position;
- - fig. 4: is a perspective view of the movement apparatus in fig. 3 in an inclined position;
- - fig. 5: is a perspective view of the movement apparatus in fig. 3 in another inclined position.

### DETAILED DESCRIPTION OF A PREFERENTIAL EMBODIMENT OF THE INVENTION

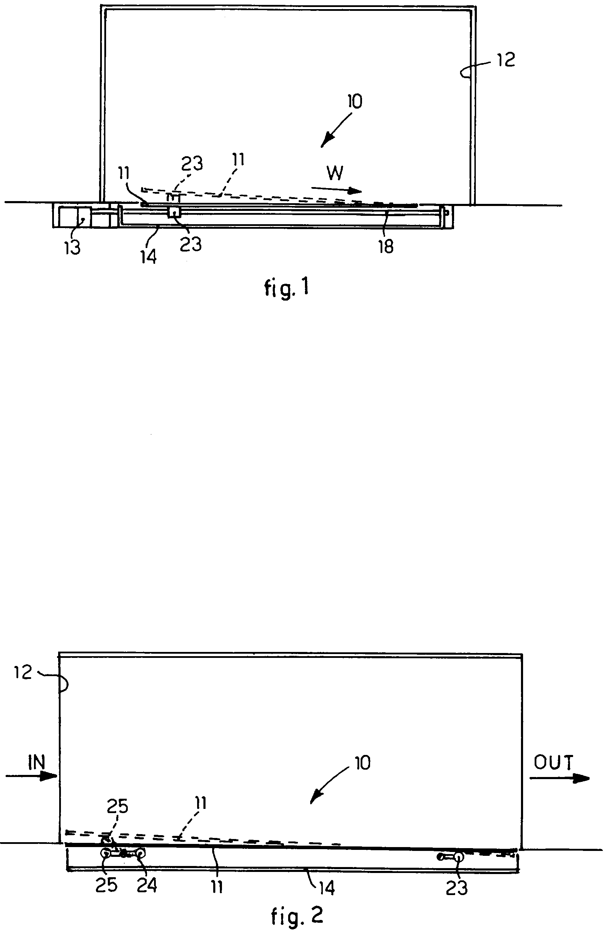

With reference to fig. 1, a movement apparatus 10 is used to move a loading plane 11 of a washing chamber 12, for washing trolley-mounted objects such as trolleys, beds, bedside tables or other equipment to be subjected to washing and heat disinfection, inside the washing chamber 12.

Inside the washing chamber 12 there is a floor structure 14, on which the loading plane 11 is disposed, in such a manner that it is not constrained to the floor 14, so that it can be inclined as desired, as will be described in detail hereafter in the description. The loading plane 11 is advantageously of a grid-like structure 15 and, in a non-restrictive embodiment, has two lanes, parallel to and equidistant from each other, to accommodate in sliding manner the object to be washed, for example a trolley; to this purpose the object is generally provided with sliding means, such as castor-type wheels.

The object can be inserted into the washing chamber 12 through an aperture in the direction of entry IN and is removed, at the end of the washing cycle, through another aperture, in the direction of exit OUT advantageously opposite the direction IN (figs. 2, 3, 4 and 5).

For the washing and heat disinfection cycle, the washing chamber 12 is provided with a hydraulic introduction system, not shown in the drawings and already known, to introduce a washing liquid, hot water and/or steam, able to be associated with suitable detergents. The washing liquid is delivered abundantly towards and onto the object to be washed; at the end of the washing cycle, the object has on its horizontal surfaces a large quantity of residual washing liquid, which has to be eliminated.

To this purpose the movement apparatus 10 comprises an inclination device 16, able to influence the loading plane 11, in order to selectively vary the inclination thereof, and consequently to vary the angular position of the object resting thereon, that is, its inclination with respect to the floor 14. In this way, by means of said inclination, the residual washing liquid falls due to gravity and is discharged from the object.

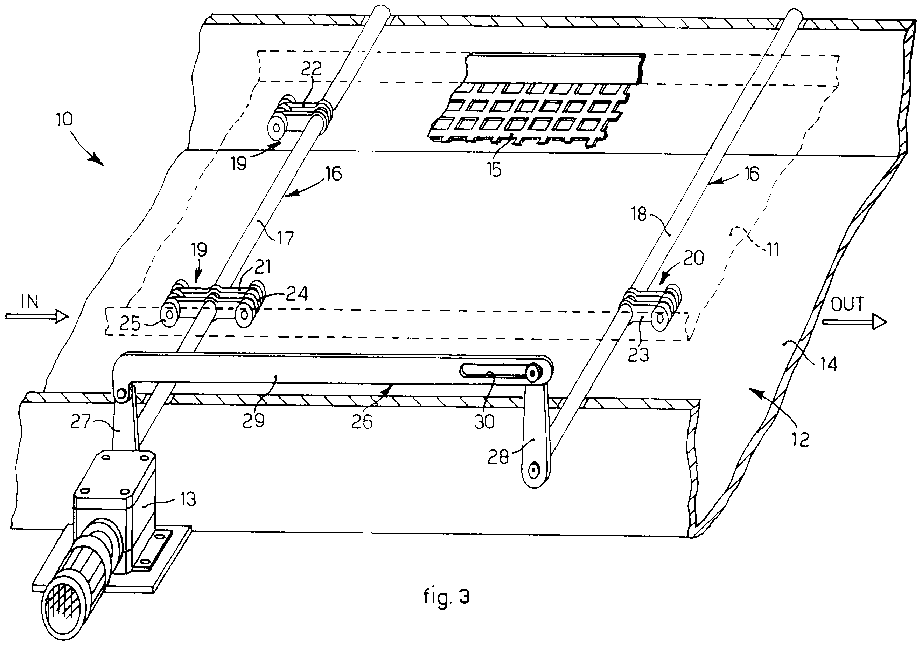

According to the present invention, the inclination device 16 comprises at least two rotation bars, able to be driven by a single actuation mean or motor 13, in order to rotate each one of the rotation bars around its own main axis and consequently influence the position of the loading plane 11, so as to incline it in at least two different directions with respect to an inactive position, wherein the loading plane 11 and hence the object are horizontal, that is, parallel to the floor 14 (fig. 3).

According to the present invention, the rotation bars are disposed inside the washing chamber between the floor 14 and the loading plane 11, they are distanced from each other over the length of the loading plane and preferably parallel to each other and moreover they are transversal, advantageously perpendicular, to the direction of entry IN and/or of exit OUT of the object.

According to the present invention, the rotation bars comprise a first rotation bar 17, substantially in proximity with the entry to the washing chamber 12, and a second rotation bar 18, substantially in proximity with the exit from the washing chamber 12 (fig. 3), parallel to each other.

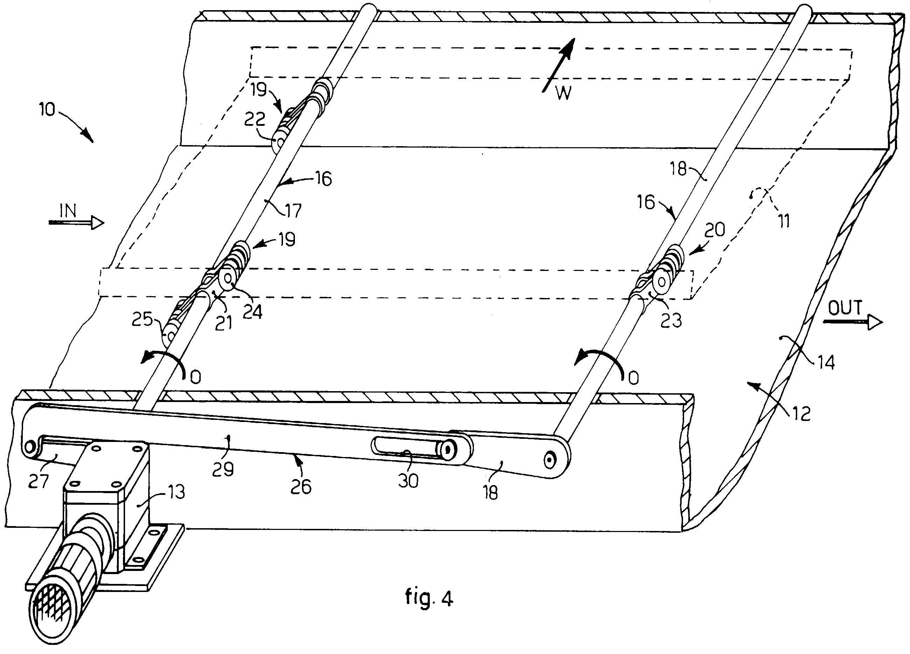

Advantageously, an exit direction W (figs. 1 and 4) is selected for the residual washing water, preferably transversal, or perpendicular to the direction of entry IN and/or of exit OUT of the object, that is, parallel to the rotation bars 17, 18 (fig. 4).

In order to selectively incline the loading plane 11, the rotation bars are provided with first and second cam means which, once the rotation bars have been made to rotate by the motor 13, are able to interfere with the loading plane 11, selectively causing the desired inclination thereof.

According to the present invention, the first rotation bar 17 has ends bearing first cam means 19 (figs. 3, 4 and 5), which are disposed substantially horizontal, that is, parallel to the loading plane 11, for example during washing, in the inactive position, that is to say, when the loading plane 11 is horizontal.

According to the present invention, moreover, the second rotation bar 18 has one end that comprises at least second cam means 20, also horizontal, at least in said inactive position.

The first cam means 19 comprise at one end of the first rotation bar 17 a double cam element 21, and at an opposite end a first single cam element 22 (figs. 3, 4 and 5). The double cam element 21 is parallel to the loading plane 11 in the inactive position, and therefore does not interfere with it in said inactive position.

The double cam element 21 has a first and a second active portion 24, 25, diametrically opposite the first rotation bar 17 and therefore disposed at a plane angle between them and orthogonal to the first rotation bar 17 itself.

The first single cam element 22 is also orthogonal to the first rotation bar 17 and extends parallel to the double cam element 21, being facing towards the entry of the washing chamber 12, in the inactive position (fig. 3).

The second cam means 20 comprise at least a second single cam element 23, disposed at one end of the second rotation bar 18, in a position diagonally opposite the first single cam element 22.

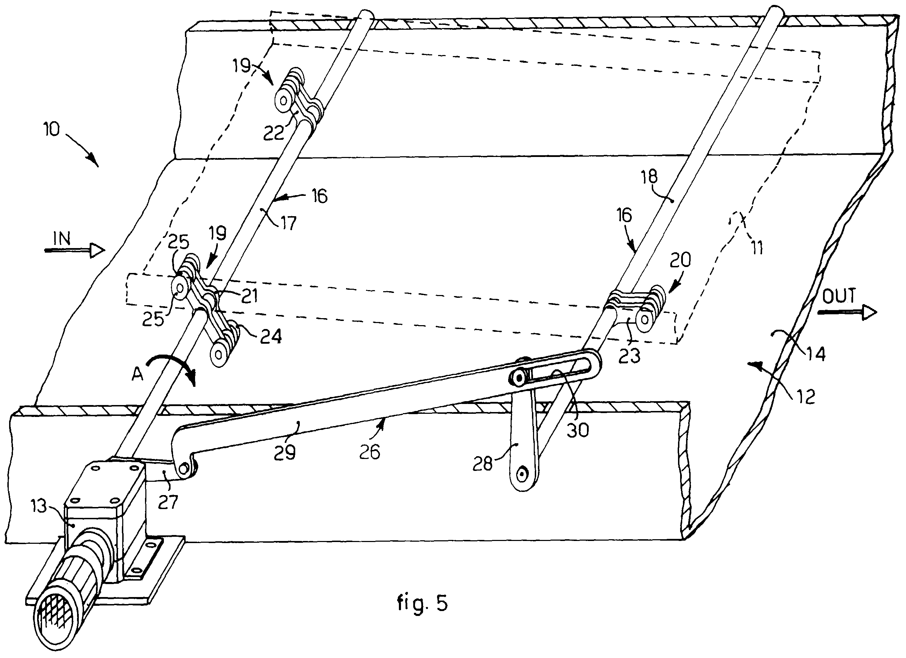

The second single cam element 23 is orthogonal to the second rotation bar 18, parallel to the double cam element 21 in the inactive position, and facing towards the exit of the washing chamber 12. Therefore, in the inactive position, the double cam element 21 and the single cam elements 22, 23 lie on the same plane, parallel to the loading plane 11, not interfering with the latter in the inactive position. When the rotation bars are made to rotate simultaneously and in synchrony, from the inactive position, in a first direction of rotation 0 (fig. 4), for example by about 90° in an anti-clockwise direction, the double cam element 21 and the second single cam element 23 are disposed in a substantially vertical position. The latter, therefore, interfere with the loading plane 11, respectively by means of the single cam element 23 and the first active portion 24, and raise it on one side for its first inclination (figs. 1 and 4) and so that the residual water is discharged, while the first single cam element 22 rotates downwards, and is disposed in a vertical position facing towards the floor 14, in the direction opposite to the loading plane 11, and therefore does not interfere with the loading plane 11. By means of an inverse rotation, the inclination device 16 is returned, from the position where it is inclined in order to discharge the water, to the inactive position. When at least the first rotation bar 17 is made to rotate, from the inactive position, in a second direction of rotation A (fig. 5), different from and opposite to said first direction of rotation, for example by 90° in a clockwise direction, the double cam element 21 and the first single cam element 22 are disposed in a substantially vertical position, interfering with the loading plane 11, in this case by means of the second active portion 25 and the single element 22, and raising it on the entry side for a second inclination (figs. 2 and 5), in a direction transverse to the first inclination and hence to facilitate the discharge of the object; in this case, by rotating only the first rotation bar 17, the second rotation bar 18 does not rotate and the second single cam element 23 is in a horizontal position, and does not interfere with the loading plane 11. Alternatively, for the second inclination in order to discharge the object, the second rotation bar 18 can rotate simultaneously and in synchrony with the first rotation bar: in this case the second single cam element 23 does not in any case interfere with the loading plane 11, because it is positioned facing downwards, in this case towards the floor 14, vertically, or in the direction opposite the loading plane 11.

By means of the disposition of the cam elements as shown above, the rotation bars are able to move and incline the loading plane 11, and hence the object, in the direction W, in order to discharge the residual washing water, by rotating the first rotation bar 17 and the second rotation bar 18 in the first direction of rotation O. Furthermore, the rotation bars are able to incline the loading plane 11, and hence the object, in the direction OUT, by rotating at least the first rotation bar 17 in the second direction of rotation A, different from the direction of rotation of the arrows O (fig. 5). In this case, as we said, the first cam means 19 act and interfere against the loading plane 11, simultaneously and in synergy, causing it to be raised on the entry side of the washing chamber 12 and hence causing the inclination towards the exit of the object, which slides outside due to gravity.

In fact, because the object is able to slide along the lanes, following this last inclination towards the exit, the object itself is made to slide outside the washing chamber, and the automatic extraction thereof is thus achieved.

Returning to the movement of the rotation bars by means of the motor 13, the latter is kinematically connected to both the rotation bars 17, 18 by means of a system of kinematic transmission 26, which comprises a first rotation lever 27, connected to the outlet of the motor 13 in order to receive a drive torque therefrom, and a second rotation lever 28, said levers 27, 28 being respectively pivoted to the first and second rotation bar 17 and 18, and moreover reciprocally connected by means of a connection bar 29 which, substantially roto-translating, transmits the torque to the second rotation lever 28. In the inactive position, the connection bar 29 is substantially horizontal, while the rotation levers 27, 28 are substantially orthogonal thereto. According to one embodiment of the present invention, the connection bar 29 has an eyelet 30, in which one end of the second rotation lever 28 is able to slide, when the connection bar 29 moves, for example forwards, in such a manner as to release the second rotation lever 27 from the motion of rotation of the first rotation lever 27 and of the connection bar 29. The connection bar 29 is moved backwards, towards the entry of the washing chamber 12, by the rotation of the first rotation lever 27, when the first inclination is achieved in order to discharge the water, and in this displacement it influences the position of the second rotation lever 28, making it rotate, and consequently makes the respective second rotation bar 18 rotate. In order to achieve the second inclination, so as to discharge the object, from the inactive position the connection bar 29 is moved forwards, that is, towards the exit, by the rotation of the first rotation lever 27. In this movement, the second rotation lever 28 remains stationary, substantially vertical, since it slides in the eyelet 30 and consequently the second rotation bar 18 does not rotate either; in this way, the second rotation lever 28 is more easily driven for subsequent rotations, preventing risks of any blockages. Alternatively, if no eyelet 30 is included, the second rotation lever 28 is made to rotate forwards by the connection bar 29 and the second rotation bar 18 consequently rotates too, however without interfering with its own cam elements with the loading plane 11.

To this purpose, the rotation of the rotation bars and the action of the motor 13 and the system of kinematic transmission 26 are automatically managed by means of commands from a control unit which, advantageously interfacing and/or integrated with the control unit of the washing chamber, recognizes for example the end of the washing cycle and the start of the drying step, which advantageously occurs inside the washing chamber 12, and consequently sends a command to the motor 13 of the rotation bars, in order to rotate the bars, for example in the direction O, so as to discharge the water, for example after the washing steps and before the drying step, and in order to subsequently re-position horizontally the loading plane 11.

Finally, having received a signal of consent for the extraction of the object, for example subordinated to the opening of an automatic exit door from the washing chamber 12, the control unit commands the motor 13 to rotate the rotation bars, for example in direction A, which allows the automatic sliding discharge of the object.

It is clear, however, that modifications and/or additions of parts may be made to the movement apparatus 10 as described heretofore, without departing from the scope of the claims.

It is also clear that, although the present invention has been described with reference to specific examples, a person of skill in the art shall certainly be able to achieve many other equivalent forms of movement apparatus 10 for planes in washing chambers, all of which shall come within the subject matter of the claims.

## Claims

1. Apparatus for moving at least a loading plane (11) for a washing chamber (12), wherein on said loading plane (11) at least an object, such as a trolley-mounted object, is able to be moved between an entry (IN) and an exit (OUT), and to rest so as to be washed by means of a washing liquid, wherein said movement apparatus comprises an inclination device (16), able to influence said loading plane (11) so as to vary the inclination thereof, in such a manner as to allow, selectively, at least to discharge the washing liquid from said object, said movement apparatus being **characterized in that** said inclination device (16) comprises:
- a first rotation bar (17) having first cam means (19);
- a second rotation bar (18) having second cam means (20); wherein said first and second rotation bar (17, 18) are able to rotate simultaneously in a first direction of rotation (0) so that the respective cam means (19, 20) interfere with said loading plane (10) and determine a first inclination thereof on one side of said loading plane (10), in order to discharge said washing liquid from the surface of said object, wherein at least said first rotation bar (17) is able to rotate in a second direction of rotation (A), different from said first direction of rotation (0), so that at least said first cam means (19) interfere with said loading plane (11) and determine a second inclination forwards thereof, in order to discharge said object from said washing chamber (12).

2. Movement apparatus as in claim 1, **characterized in that** said inclination device (16) comprises a single motor (13), able to move in rotation said first rotation bar (17) and said second rotation bar (18) by means of a system of kinematic transmission (26), in order to selectively achieve said first inclination and said second inclination.

3. Movement apparatus as in any claim hereinbefore, **characterized in that** said first rotation bar (17) and said second rotation bar (18) are positioned parallel to each other in said washing chamber (12).

4. Movement apparatus as in any claim hereinbefore, **characterized in that** it comprises a control unit, able to detect the end of said wash and to command said motor (13) in order to allow, selectively and automatically, to discharge the washing liquid and to discharge said object from said washing chamber (12).

5. Washing chamber, **characterized in that** it comprises a movement apparatus as in any of the previous claims and a loading plane (11), wherein said loading plane (11) has at least two lanes in which said object slides, disposed substantially in the direction of entry and/or exit of said washing chamber, said lanes being able to allow said object to slide and be discharged from said washing chamber.

## Patentansprüche

1. Vorrichtung zum Bewegen mindestens einer Ladeebene (11) für eine Waschkammer (12), wobei auf der Ladeebene (11) mindestens ein Gegenstand, wie ein auf einem Transportkarren angebrachter Gegenstand, zwischen einem Eingang (IN) und einem Ausgang (OUT) bewegt werden kann und bleiben kann, um mittels einer Waschflüssigkeit gewaschen zu werden, wobei die Bewegungsvorrichtung eine Neigungsvorrichtung (16) aufweist, die in der Lage ist, die Ladeebene (11) so zu beeinflussen, dass deren Neigung geändert wird, so dass selektiv zumindest das Ablaufen lassen der Waschflüssigkeit von dem Gegenstand ermöglicht wird, wobei die Bewegungsvorrichtung **dadurch gekennzeichnet ist, dass** die Neigungsvorrichtung (16) Folgendes umfasst:
- eine erste Drehstange (17) mit ersten Nockenmitteln (19);
- eine zweite Drehstange (18) mit zweiten Nockenmitteln (20);
wobei die erste und die zweite Drehstange (17, 18) in der Lage sind, sich gleichzeitig in eine erste Drehrichtung (O) zu drehen, so dass die jeweiligen Nockenmittel (19, 20) in die Ladeebene (10) eingreifen und eine erste Neigung von dieser auf einer Seite der Ladeebene (10) bestimmen, um die Waschflüssigkeit von der Oberfläche des Gegenstands ablaufen zu lassen, wobei mindestens die erste Drehstange (17) in der Lage ist, sich in eine zweite Drehrichtung (A) zu drehen, die sich von der ersten Drehrichtung (O) unterscheidet, so dass mindestens die ersten Nockenmittel (19) in die Ladeebene (11) eingreifen und eine zweite Neigung nach vorne bestimmen, um den Gegenstand aus der Waschkammer (12) auszugeben.

2. Bewegungsvorrichtung nach Anspruch 1, **dadurch gekennzeichnet, dass** die Neigungsvorrichtung (16) einen einzelnen Motor (13) umfasst, der in der Lage ist, die erste Drehstange (17) und die zweite Drehstange (18) mittels eines Systems kinematischer Übertragung (26) drehend zu bewegen, um die erste Neigung und die zweite Neigung selektiv zu erreichen.

3. Bewegungsvorrichtung nach einem der vorhergehenden Ansprüche, **dadurch gekennzeichnet, dass** die erste Drehstange (17) und die zweite Drehstange (18) parallel zueinander in der Waschkammer (12) positioniert sind.

4. Bewegungsvorrichtung nach einem der vorhergehenden Ansprüche, **dadurch gekennzeichnet, dass** sie eine Steuereinheit umfasst, die in der Lage ist, das Ende des Waschens zu erkennen und dem Motor (13) Befehle zu geben, um zu ermöglichen, die Waschflüssigkeit selektiv und automatisch ablaufen zu lassen und den Gegenstand aus der Waschkammer (12) auszugeben.

5. Waschkammer, **dadurch gekennzeichnet, dass** sie eine Bewegungsvorrichtung nach einem der vorhergehenden Ansprüche sowie eine Ladeebene (11) aufweist, wobei die Ladeebene (11) mindestens zwei Spuren aufweist, in denen der Gegenstand gleitet, im Wesentlichen in Richtung des Eingangs und/oder Ausgangs der Waschkammer angeordnet, wobei die Spuren in der Lage sind, dem Gegenstand ein Gleiten und die Ausgabe aus der Waschkammer zu ermöglichen.

## Revendications

1. Appareil destiné à déplacer au moins un plan de chargement (11) pour une chambre de lavage (12), dans lequel sur ledit plan de chargement (11), au moins un objet, tel qu'un objet monté sur chariot, est apte à être déplacé entre une entrée (IN) et une sortie (OUT), et à être en appui de manière à être lavé au moyen d'un liquide de lavage, dans lequel ledit appareil de déplacement comporte un dispositif d'inclinaison (16), apte à influencer ledit plan de chargement (11) de manière à faire varier l'inclinaison de celui-ci, de telle manière à permettre, sélectivement, au moins d'évacuer le liquide de lavage dudit objet, ledit appareil de déplacement étant **caractérisé en ce que** ledit dispositif d'inclinaison (16) comporte :
- une première barre de rotation (17) ayant des premiers moyens de came (19),
- une seconde barre de rotation (18) ayant des seconds moyens de came (20), dans lequel lesdites première et seconde barres de rotation (17, 18) sont aptes à tourner simultanément dans un premier sens de rotation (O) de sorte que les moyens de came respectifs (19, 20) interfèrent avec ledit plan de chargement (10) et déterminent une première inclinaison de celui-ci sur un côté dudit plan de chargement (10), afin d'évacuer ledit liquide de lavage de la surface dudit objet, dans lequel au moins ladite première barre de rotation (17) est apte à tourner dans un second sens de rotation (A), différent dudit premier sens de rotation (O), de sorte qu'au moins lesdits premiers moyens de came (19) interfèrent avec ledit plan de chargement (11) et déterminent une second inclinaison vers l'avant de celui-ci, afin de décharger ledit objet de ladite chambre de lavage (12).

2. Appareil de déplacement selon la revendication 1, **caractérisé en ce que** ledit dispositif d'inclinaison (16) comporte un moteur unique (13), apte à mettre en rotation ladite première barre de rotation (17) et ladite seconde barre de rotation (18) au moyen d'un système de transmission cinématique (26), afin d'obtenir sélectivement ladite première inclinaison et ladite seconde inclinaison.

3. Appareil de déplacement selon l'une quelconque des revendications ci-dessus, **caractérisé en ce que** ladite première barre de rotation (17) et ladite seconde barre de rotation (18) sont positionnées parallèles l'une à l'autre dans ladite chambre de lavage (12).

4. Appareil de déplacement selon l'une quelconque des revendications ci-dessus, **caractérisé en ce qu'**il comporte une unité de commande, apte à détecter la fin dudit lavage et à commander ledit moteur (13) afin de permettre, sélectivement et automatiquement, d'évacuer le liquide de lavage et de décharger ledit objet de ladite chambre de lavage (12).

5. Chambre de lavage, **caractérisée en ce qu'**elle comporte un appareil de déplacement selon l'une quelconque des revendications précédentes et un plan de chargement (11), dans laquelle ledit plan de chargement (11) a au moins deux voies dans lesquelles ledit objet glisse, disposées substantiellement dans la direction d'entrée et/ou de sortie de ladite chambre de lavage, lesdites voies étant aptes à permettre audit objet de glisser et d'être déchargé de ladite chambre de lavage.
